# EUROPEAN PATENT APPLICATION

(11) **EP 4 671 234 A1**
(43) Date of publication of application: **31.12.2025**
(21) Application number: 25185586.2
(22) Date of filing: 26.06.2025
(51) Int. Cl.: C07C 249/12, C07C 251/38

(54) **METHOD AND APPARATUS FOR PREPARING ETHYL (Z)-2-METHOXYIMINO-3-OXOBUTANOATE**

(30) Priority: 27.06.2024 CN 202410845625
(71) Applicant: Shandong Heyi Gas Co., Ltd., Dongying City, Shandong Province 257500 (CN)
(72) Inventor: YU, Chunyu, Dongying City, Shandong Province, 257500 (CN); LI, Kangning, Dongying City, Shandong Province, 257500 (CN); ZHANG, Shujiang, Dongying City, Shandong Province, 257500 (CN); YANG, Jun, Dongying City, Shandong Province, 257500 (CN); XU, Baoyun, Dongying City, Shandong Province, 257500 (CN); WANG, Zhejun, Dongying City, Shandong Province, 257500 (CN); YAN, Xiuxiang, Dongying City, Shandong Province, 257500 (CN)
(74) Representative: Schneiders & Behrendt Bochum

(57) **Abstract**

Disclosed are a method and an apparatus for preparing ethyl (Z)-2-methoxyimino-3-oxobutanoate, which belongs to the technical field of organic synthesis. The method for preparing ethyl (Z)-2-methoxyimino-3-oxobutanoate does not require the use of an acid scavenger and a phase transfer catalyst, and includes: mixing ethyl 2-(hydroxyimino)acetoacetate, methanol, a concentrated aqueous sulfuric acid solution, and a boron trifluoride-methanol complex, and subjecting a resulting mixture to esterification-methylation reaction to obtain the ethyl (Z)-2-methoxyimino-3-oxobutanoate. In the method, dimethyl sulfate is directly generated in a reaction system and further undergoes methylation reaction, without using the acid scavenger and the phase transfer catalyst, with simple operations and lower cost.

## Description

### TECHNICAL FIELD

The present disclosure relates to the technical field of organic synthesis, and in particular to a method and an apparatus for preparing ethyl (Z)-2-methoxyimino-3-oxobutanoate.

### BACKGROUND ART

AE active ester (MAEM), with a chemical name of 2-mercaptobenzothiazolyl (Z)-2-(2-aminothiazole-4-yl)-2-methoxyimino acetate, constitutes an important side chain of cephalosporin antibiotics. MAEM can be used to produce ceftriaxone, cefotaxime, cefetamet pivoxil and other pioneer drugs. MAEM is one of the pharmaceutical intermediates with good development prospects and high added value, since cephalosporins have the characteristics of high efficiency, low toxicity and broad antibacterial spectrum.

At present, MAEM is synthesized generally by first synthesizing 2-(2-aminothiazole-4-yl)-2-methoxyiminoacetic acid through oximation, methylation, chlorination, cyclization, hydrolysis and other steps using ethyl acetoacetate as the starting material, and then condensing with 2,2'-dithiobis(benzothiazole) to obtain the target product. The traditional methylation reaction usually uses dimethyl sulfate as the methylating agent, and occurs in the presence of a base in a large amount as an acid scavenger and a phase transfer catalyst. Dimethyl sulfate is a highly toxic compound, has a similar effect as mustard gas, but has toxicity 15 times greater than that of chlorine gas, which makes it difficult to operate and control the feeding process. In addition, dimethyl sulfate decomposes into sulfuric acid and methanol when encountering water during storage, which is difficult to store; there is also a problem of a high price of the phase transfer catalyst used.

### SUMMARY

An object of the present disclosure is to provide a method and an apparatus for preparing ethyl (Z)-2-methoxyimino-3-oxobutanoate. In the method according to the present disclosure, dimethyl sulfate is directly generated in a reaction system and further undergoes methylation reaction, without using an acid scavenger and a phase transfer catalyst, with simple operations and lower cost.

To achieve the above object, the present disclosure provides the following technical solutions:
Provided is a method for preparing ethyl (Z)-2-methoxyimino-3-oxobutanoate, without using an acid scavenger and a phase transfer catalyst, including:
mixing ethyl 2-(hydroxyimino)acetoacetate, methanol, a concentrated aqueous sulfuric acid solution (also referred to as concentrated sulfuric acid), and a boron trifluoride-methanol complex, and subjecting a resulting mixture to esterification-methylation reaction to obtain the ethyl (Z)-2-methoxyimino-3-oxobutanoate.

In the present disclosure, the boron trifluoride-methanol complex has a molecular formula of BF₃·CH₃OH, with CAS No. 373-57-9.

In some embodiments, the mixing includes: subjecting the ethyl 2-(hydroxyimino)acetoacetate and the methanol to primary stirring to obtain a primary mixed solution; and
dropwise adding the concentrated aqueous sulfuric acid solution and the boron trifluoride-methanol complex to the primary mixed solution.

In some embodiments, the concentrated aqueous sulfuric acid solution is added dropwise at a dripping rate of 20-30 g/h, and the boron trifluoride-methanol complex is added dropwise at a dripping rate of 6-10 g/h; and dropwise adding the concentrated aqueous sulfuric acid solution and the boron trifluoride-methanol complex is performed by starting simultaneously dripping addition of the concentrated aqueous sulfuric acid solution and the boron trifluoride-methanol complex.

In some embodiments, the concentrated aqueous sulfuric acid solution has a concentration of 95-98 wt%; a molar ratio of sulfuric acid in the concentrated aqueous sulfuric acid solution to the ethyl 2-(hydroxyimino)acetoacetate ranges from 0.9 : 1 to 2 : 1; and
a molar ratio of the ethyl 2-(hydroxyimino)acetoacetate, the methanol, and the boron trifluoride-methanol complex is in a range of 1 : 10-15 : 0.2-0.5.

In some embodiments, the esterification-methylation reaction is conducted at a temperature of 20-40 °C for 2-4 hours.

In some embodiments, the esterification-methylation reaction is conducted under stirring, and the stirring is conducted at a stirring speed of 90-150 r/min.

In some embodiments, the method further includes: after the esterification-methylation reaction, mixing a reaction mixture obtained from the esterification-methylation reaction with a saturated sodium carbonate solution, subjecting a resulting liquid mixture to a first separation for removing methanol, collecting a residue obtained from the first separation and subjecting the residue to extraction with dichloromethane, and collecting an organic phase obtained from the extraction and subjecting the organic phase to a second separation for removing dichloromethane to obtain the ethyl (Z)-2-methoxyimino-3-oxobutanoate.

In some embodiments, the first separation is performed by distillation, and the distillation is conducted at a temperature of 40-50 °C; and the method further includes: after the distillation, collecting the methanol removed by the distillation, and reusing the methanol in the esterification-methylation reaction.

In some embodiments, the second separation is performed by concentration, the concentration being reduced-pressure concentration; and the method further includes: after the concentration, collecting the dichloromethane removed by the concentration, and reusing the dichloromethane in the extraction.

Provided is an apparatus for preparing ethyl (Z)-2-methoxyimino-3-oxobutanoate, including:
a methanol storage tank (4); a dichloromethane storage tank (5); an esterification-methylation reactor (1), a first separation device (2), and a second separation device (3), communicated sequentially, where the esterification-methylation reactor (1) is provided with an ethyl 2-(hydroxyimino)acetoacetate feed inlet, a methanol feed inlet, a concentrated aqueous sulfuric acid solution feed inlet, a boron trifluoride-methanol complex feed inlet, and a reaction liquid discharge outlet;
the first separation device (2) is provided with a saturated sodium carbonate solution feed inlet, a dichloromethane feed inlet, a methanol discharge outlet, an organic phase discharge outlet, and a reaction liquid feed inlet that is communicated with the reaction liquid discharge outlet of the esterification-methylation reactor (1) through a pipeline;
the second separation device (3) is provided with a product discharge outlet, a dichloromethane discharge outlet, and an organic phase feed inlet that is communicated with the organic phase discharge outlet of the first separation device (2) through a pipeline;
the methanol storage tank (4) is provided with a feed inlet and a discharge outlet, the feed inlet of the methanol storage tank (4) is communicated with the methanol discharge outlet of the first separation device (2) through a pipeline, and the discharge outlet of the methanol storage tank (4) is communicated with the methanol feed inlet of the esterification-methylation reactor (1) through a pipeline; and
the dichloromethane storage tank (5) is provided with an inlet and an outlet, the inlet of the dichloromethane storage tank (5) is communicated with the dichloromethane discharge outlet of the second separation device (3) through a pipeline, and the outlet of the dichloromethane storage tank (5) is communicated with the dichloromethane feed inlet of the first separation device (2).

The present disclosure provides a method for preparing ethyl (Z)-2-methoxyimino-3-oxobutanoate, without using an acid scavenger and a phase transfer catalyst, includingmixing ethyl 2-(hydroxyimino)acetoacetate, methanol, a concentrated aqueous sulfuric acid solution, and a boron trifluoride-methanol complex, and subjecting a resulting mixture to esterification-methylation reaction to obtain the ethyl (Z)-2-methoxyimino-3-oxobutanoate. In the present disclosure, the boron trifluoride-methanol complex is used as a catalyst to catalyze the esterification reaction between the methanol and the concentrated aqueous sulfuric acid solution, generating dimethyl sulfate. The generated dimethyl sulfate and the ethyl 2-(hydroxyimino)acetoacetate undergo methylation reaction, thereby obtaining the target product, i.e., the ethyl (Z)-2-methoxyimino-3-oxobutanoate. Compared with the methods using the highly toxic dimethyl sulfate as a methylating agent, the method according to the present disclosure is simpler in operation and safer; moreover, the method according to the present disclosure does not require the use of an acid scavenger or a phase transfer catalyst, which helps to reduce costs.

Furthermore, reaction conditions in the method according to the present disclosure are mild, with few side reactions, high product yield, and high purity. The methanol and dichloromethane used could be recycled, which significantly reduces environmental pollution while effectively lowers production costs, resulting in higher economic benefits.

### BRIEF DESCRIPTION OF THE DRAWINGS

FIG. 1 shows a schematic diagram of an apparatus for preparing ethyl (Z)-2-methoxyimino-3-oxobutanoate in an embodiment according to the present disclosure, where 1 represents an esterification-methylation reactor, 2 represents a first separation device, 3 represents a second separation device, 4 represents a methanol storage tank, and 5 represents a dichloromethane storage tank.
FIG. 2 shows a flowchart of the method for preparing ethyl (Z)-2-methoxyimino-3-oxobutanoate in an example of the present disclosure.

### DETAILED DESCRIPTION OF THE EMBODIMENTS

In the present disclosure, unless otherwise specified, all raw materials used are commercially available goods well known to those skilled in the art or prepared by methods well known to those skilled in the art.

The present disclosure provides a method for preparing ethyl (Z)-2-methoxyimino-3-oxobutanoate, without using an acid scavenger and a phase transfer catalyst, including/consisting of:
mixing ethyl 2-(hydroxyimino)acetoacetate, methanol, a concentrated aqueous sulfuric acid solution, and a boron trifluoride-methanol complex, and subjecting a resulting mixture to esterification-methylation reaction to obtain the ethyl (Z)-2-methoxyimino-3-oxobutanoate.

In the present disclosure, ethyl 2-(hydroxyimino)acetoacetate, methanol, the concentrated aqueous sulfuric acid solution, and the boron trifluoride-methanol complex are mixed, and a resulting mixture is subjected to esterification-methylation reaction to obtain the ethyl (Z)-2-methoxyimino-3-oxobutanoate. In some embodiments of the present disclosure, a molar ratio of the ethyl 2-(hydroxyimino)acetoacetate to the methanol is in a range of 1 : 10-15, specifically 1 : 10, 1 : 11, 1 : 12, 1 : 13, 1 : 14 or 1 : 15; a molar ratio of the ethyl 2-(hydroxyimino)acetoacetate to the boron trifluoride-methanol complex is preferably in a range of 1 : 0.2-0.5, specifically 1 : 0.2, 1 : 0.25, 1 : 0.3, 1 : 0.35, 1 : 0.4, 1 : 0.45 or 1 : 0.5. In some embodiments of the present disclosure, the concentrated aqueous sulfuric acid solution has a concentration of 95-98 wt%; a molar ratio of sulfuric acid in the concentrated aqueous sulfuric acid solution to ethyl 2-(hydroxyimino)acetoacetate is preferably in a range of 0.9-2 : 1, more preferably 0.9 : 1, 1 : 1, 1.2 : 1, 1.5 : 1, 1.8 : 1 or 2 : 1.

In some embodiments of the present disclosure, the mixing includes: subjecting the ethyl 2-(hydroxyimino)acetoacetate and the methanol to primary mixing to obtain a primary mixed solution; and dropwise adding the concentrated aqueous sulfuric acid solution and the boron trifluoride-methanol complex to the primary mixed solution. In the present disclosure, there is no special limitation on the primary mixing, as long as the thorough mixing could be realized. In the present disclosure, the temperature of the primary mixed solution is raised to the reaction temperature of esterification-methylation reaction, and the concentrated aqueous sulfuric acid solution and the boron trifluoride-methanol complex are added dropwise into the primary mixed solution; the concentrated aqueous sulfuric acid solution is added dropwise at a dripping rate of preferably 20-30 g/h, more preferably 25 g/h; the boron trifluoride-methanol complex is added dropwise at a dripping rate of preferably 6-10 g/h, more preferably 8 g/h; dropwise adding the concentrated aqueous sulfuric acid solution and the boron trifluoride-methanol complex is performed by starting simultaneously dripping addition of the concentrated aqueous sulfuric acid solution and the boron trifluoride-methanol complex. In some embodiments of the present disclosure, the concentrated aqueous sulfuric acid solution and the boron trifluoride-methanol complex are placed in different constant pressure dropping funnels, and the concentrated aqueous sulfuric acid solution and the boron trifluoride-methanol complex are then dripped simultaneously into the primary mixed solution. In some embodiments of the present disclosure, the adding is conducted under stirring; the stirring is conducted at a stirring speed of preferably 100-150 r/min, and more preferably 120 r/min.

In some embodiments of the present disclosure, the esterification-methylation reaction is conducted at a temperature of 20-40 °C, specifically 20 °C, 25 °C, 30 °C, 35 °C, or 40 °C; the esterification-methylation reaction is conducted for preferably 2-4 h, and more preferably 2-3 h; the reaction time of the esterification-methylation reaction is calculated starting from the completion of the dripping addition of the concentrated aqueous sulfuric acid solution and the boron trifluoride-methanol complex. In some embodiments of the present disclosure, the esterification-methylation reaction is conducted under stirring; the stirring is preferably conducted at a stirring speed of 100-150 r/min, and more preferably 120 r/min.

In some embodiments of the present disclosure, the method further includes: after the esterification-methylation reaction, mixing a reaction mixture obtained from the esterification-methylation reaction with a saturated sodium carbonate solution, subjecting a resulting liquid mixture to a first separation for removing methanol, collecting a residue obtained from the first separation and subjecting the residue to extraction with dichloromethane, and collecting an organic phase obtained from the extraction and subjecting the organic phase to a second separation for removing dichloromethane to obtain the ethyl (Z)-2-methoxyimino-3-oxobutanoate. In the present disclosure, the saturated sodium carbonate solution plays the role of quenching BF₃. In some embodiments of the present disclosure, the first separation is performed by distillation; the distillation is conducted at a temperature of preferably 40-50 °C, and more preferably 45 °C; the distillation also obtains methanol, and the method further preferably includes: after the distillation, collecting the methanol obtained from the distillation, and reusing the methanol collected in the esterification-methylation reaction. In some embodiments of the present disclosure, the second separation is performed by concentration; the concentration is preferably reduced-pressure concentration; the concentration also obtains dichloromethane, and the method preferably further includes: after the concentration, collecting the dichloromethane obtained from the concentration, and reusing the dichloromethane collected in the extraction.

The present disclosure also provides an apparatus for preparing ethyl (Z)-2-methoxyimino-3-oxobutanoate, including/consisting of: a methanol storage tank; a dichloromethane storage tank; and an esterification-methylation reactor, a first separation device, and a second separation device sequentially communicated; where
the esterification-methylation reactor is provided with an ethyl 2-(hydroxyimino)acetoacetate feed inlet, a methanol feed inlet, a concentrated aqueous sulfuric acid solution feed inlet, a boron trifluoride-methanol complex feed inlet, and a reaction liquid discharge outlet;
the first separation device is provided with a saturated sodium carbonate solution feed inlet, a dichloromethane feed inlet, a methanol discharge outlet, an organic phase discharge outlet, and a reaction liquid feed inlet that is communicated with the reaction liquid discharge outlet through a pipeline;
the second separation device is provided with a product discharge outlet, a dichloromethane discharge outlet, and an organic phase feed inlet that is communicated with the organic phase discharge outlet through a pipeline;
the methanol storage tank is provided with a feed inlet and a discharge outlet, the feed inlet of the methanol storage tank is communicated with the methanol discharge outlet of the first separation device through a pipeline, and the discharge outlet of the methanol storage tank is communicated with the methanol feed inlet of the esterification-methylation reactor through a pipeline; and
the dichloromethane storage tank is provided with an inlet and an outlet, the inlet of the dichloromethane storage tank is communicated with the dichloromethane discharge outlet of the second separation device through a pipeline, and the outlet of the dichloromethane storage tank is communicated with the dichloromethane feed inlet of the first separation device.

In the present disclosure, there is no special limitation on the specific dimensions and materials of the methanol storage tank, the dichloromethane storage tank, the esterification-methylation reactor, the first separation device, and the second separation device, as long as the practical production requirements are met according to the method for preparing ethyl (Z)-2-methoxyimino-3-oxobutanoate in technical solutions as described above.

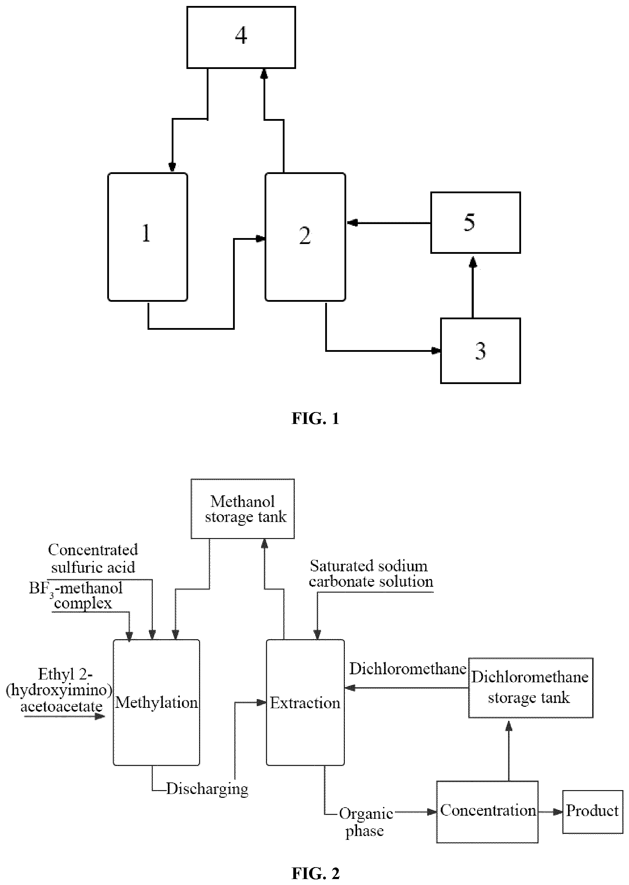

FIG. 1 shows a schematic diagram of an apparatus for preparing ethyl (Z)-2-methoxyimino-3-oxobutanoate in an embodiment according to the present disclosure, where 1 represents an esterification-methylation reactor, 2 represents a first separation device, 3 represents a second separation device, 4 represents a methanol storage tank, and 5 represents a dichloromethane storage tank; FIG. 2 shows a flowchart of the method for preparing ethyl (Z)-2-methoxyimino-3-oxobutanoate in an example of the present disclosure. The technical solutions of the present disclosure will be clearly and completely described below in conjunction with the examples of the present disclosure. Obviously, the described examples are only part of but not all of the examples of the present disclosure. Based on the examples of the present disclosure, all other examples obtained by those skilled in the art without creative labour should fall within the scope of the present disclosure.

The concentrated aqueous sulfuric acid solution used in the following examples and comparative example had a concentration of 98 wt%.

### Example 1

159.05 g of ethyl 2-(hydroxyimino)acetoacetate was mixed with 373.22 g of anhydrous methanol, and the resulting mixed solution was heated to a temperature of 35 °C. 29.94 g of a BF₃-methanol complex and 98.15 g of the concentrated aqueous sulfuric acid solution were separately placed in different constant pressure dropping funnels; at a stirring speed of 120 r/min, they were dropwise added simultaneously to the resulting mixed solution, where the dripping rate of the BF₃-methanol complex was 8 g/h, and the dripping rate of the concentrated aqueous sulfuric acid solution was 25 g/h. After the dripping addition, the resulting mixture was maintained at the temperature and subjected to reaction for 2 h.

After the reaction, 50 mL of saturated sodium carbonate solution was added to the resulting reaction system to quench BF₃, and the resulting reaction mixture was subjected to distillation at 45 °C to remove excess methanol. 200 mL of dichloromethane was added to the residue obtained from the distillation and the extraction was conducted, and an organic phase obtained from the extraction was collected and then subjected to reduced-pressure concentration to obtain 152.8 g of ethyl (Z)-2-methoxyimino-3-oxobutanoate, with a yield of 88.34%.

### Example 2

159.05 g of ethyl 2-(hydroxyimino)acetoacetate was mixed with 373.22 g of anhydrous methanol, and the resulting mixed solution was heated to a temperature of 25 °C. 29.94 g of a BF₃-methanol complex and 98.04 g of the concentrated aqueous sulfuric acid solution were separately placed in different constant pressure dropping funnels; at a stirring speed of 120 r/min, they were dropwise added simultaneously to the resulting mixed solution, where the dripping rate of the BF₃-methanol complex was 8 g/h, and the dripping rate of the concentrated aqueous sulfuric acid solution was 25 g/h. After the dripping addition, the resulting mixture was maintained at the temperature and subjected to reaction for 2 h.

After the reaction, 50 mL of saturated sodium carbonate solution was added to the resulting reaction system to quench BF₃, and the resulting reaction mixture was subjected to distillation at 45 °C to remove excess methanol. 200 mL of dichloromethane was added to the residue obtained from the distillation and the extraction was conducted, and an organic phase obtained from the extraction was collected and then subjected to reduced-pressure concentration to obtain 159.3 g of ethyl (Z)-2-methoxyimino-3-oxobutanoate, with a yield of 92.14%.

### Example 3

159.05 g of ethyl 2-(hydroxyimino)acetoacetate was mixed with 366.04 g of anhydrous methanol, and the resulting mixed solution was heated to a temperature of 35 °C. 49.9 g of a BF₃-methanol complex and 98.02 g of the concentrated aqueous sulfuric acid solution were separately placed in different constant pressure dropping funnels; at a stirring speed of 120 r/min, they were dropwise added simultaneously to the resulting mixed solution, where the dripping rate of the BF₃-methanol complex was 8 g/h, and the dripping rate of the concentrated aqueous sulfuric acid solution was 25 g/h. After the dripping addition, the resulting mixture was maintained at the temperature and subjected to reaction for 2 h.

After the reaction, 50 mL of saturated sodium carbonate solution was added to the resulting reaction system to quench BF₃, and the resulting reaction mixture was subjected to distillation at 45 °C to remove excess methanol. 200 mL of dichloromethane was added to the residue obtained from the distillation and the extraction was conducted, and an organic phase obtained from the extraction was collected and then subjected to reduced-pressure concentration to obtain 154.3 g of ethyl (Z)-2-methoxyimino-3-oxobutanoate, with a yield of 89.23%.

### Example 4

159.05 g of ethyl 2-(hydroxyimino)acetoacetate was mixed with 366.04 g of anhydrous methanol, and the resulting mixed solution was heated to a temperature of 25 °C. 49.9 g of a BF₃-methanol complex and 98.10 g of the concentrated aqueous sulfuric acid solution were separately placed in different constant pressure dropping funnels; at a stirring speed of 120 r/min, they were dropwise added simultaneously to the resulting mixed solution, where the dripping rate of the BF₃-methanol complex was 8 g/h, and the dripping rate of the concentrated aqueous sulfuric acid solution was 25 g/h. After the dripping addition, the resulting mixture was maintained at the temperature and subjected to reaction for 2 h.

After the reaction, 50 mL of saturated sodium carbonate solution was added to the resulting reaction system to quench BF₃, and the resulting reaction mixture was subjected to distillation at 45 °C to remove excess methanol. 200 mL of dichloromethane was added to the residue obtained from the distillation and the extraction was conducted, and an organic phase obtained from the extraction was collected and then subjected to reduced-pressure concentration to obtain 156.7 g of ethyl (Z)-2-methoxyimino-3-oxobutanoate, with a yield of 90.55%.

### Example 5

159.05 g of ethyl 2-(hydroxyimino)acetoacetate was mixed with 377.25 g of anhydrous methanol, and the resulting mixed solution was heated to a temperature of 25 °C. 29.94 g of a BF₃-methanol complex and 196.21 g of the concentrated aqueous sulfuric acid solution were separately placed in different constant pressure dropping funnels; at a stirring speed of 120 r/min, they were dropwise added simultaneously to the resulting mixed solution, where the dripping rate of the BF₃-methanol complex was 8 g/h, and the dripping rate of the concentrated aqueous sulfuric acid solution was 25 g/h. After the dripping addition, the resulting mixture was maintained at the temperature and subjected to reaction for 2 h.

After the reaction, 50 mL of saturated sodium carbonate solution was added to the resulting reaction system to quench BF₃, and the resulting reaction mixture was subjected to distillation at 45 °C to remove excess methanol. 200 mL of dichloromethane was added to the residue obtained from the distillation and the extraction was conducted, and an organic phase obtained from the extraction was collected and then subjected to reduced-pressure concentration to obtain 148.2 g of ethyl (Z)-2-methoxyimino-3-oxobutanoate, with a yield of 85.3%.

### Comparative Example 1

159.05 g of ethyl 2-(hydroxyimino)acetoacetate was mixed with 366.04 g of anhydrous methanol, and the resulting mixed solution was heated to a temperature of 25 °C. 98.17 g of the concentrated aqueous sulfuric acid solution was placed in a constant pressure dropping funnel; at a stirring speed of 120 r/min, the concentrated aqueous sulfuric acid solution was dropwise added to the resulting mixed solution at a dripping rate of 25 g/h. After the dripping addition, the resulting mixture was maintained at the temperature and subjected to reaction for 2 h.

After the reaction, 50 mL of saturated sodium carbonate solution was added to the resulting reaction system, and the resulting reaction mixture was subjected to distillation at 45 °C to remove excess methanol. 200 mL of dichloromethane was added to the residue obtained from the distillation and the extraction was conducted, and an organic phase obtained from the extraction was collected and then subjected to reduced-pressure concentration to obtain 59.2 g of ethyl (Z)-2-methoxyimino-3-oxobutanoate, with a yield of 34.2%.

Table 1 shows the relevant reaction conditions and the mass, yield, and purity data of the product in each example and comparative example. The results of the examples show that reaction temperature affect the yield and purity of the product to a certain degree; the reaction temperature should not be too high, with an optimal temperature of 25 °C. The BF₃-methanol complex is used as a catalyst, and the amount thereof used should not be excessive, which is conducive to provide a mild reaction. The amount of the concentrated aqueous sulfuric acid solution used should also not be excessive, and otherwise the excessive amount would lead to reduced product yield and difficulty in disposing residual waste acid. Additionally, from the results of Comparative Example 1 shown in Table 1, if the BF₃-methanol complex is not used, the product yield significantly decreases, and the product purity is lower.

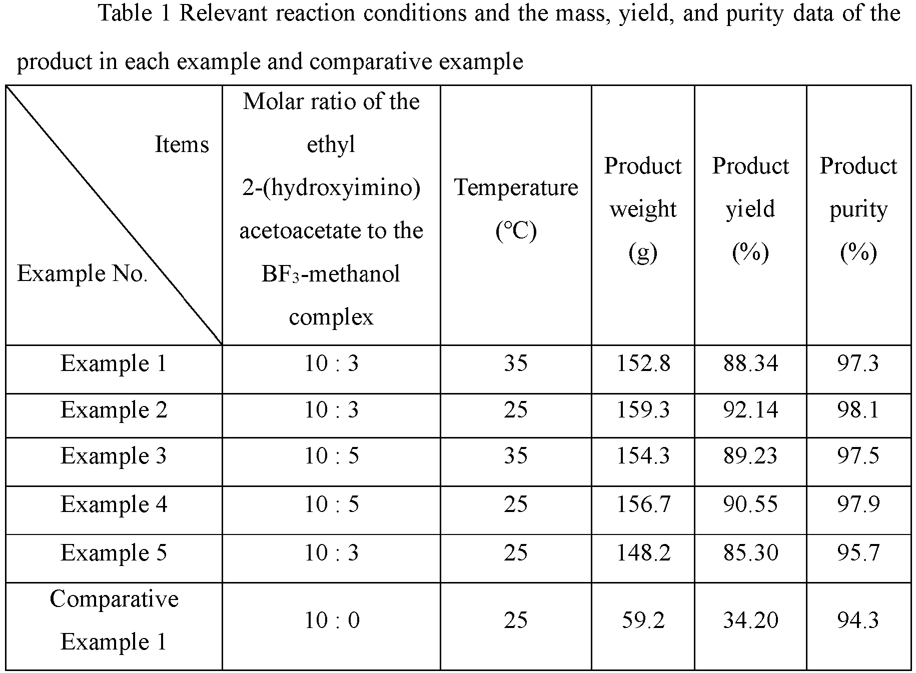

The above description is only a preferred embodiment of the present disclosure. It should be noted that, for those skilled in the art, a number of modifications and improvements could be made without departing from the principle of the present disclosure, and such modifications and improvements should also be deemed as falling within the scope of the present disclosure.

## Claims

1. A method for preparing ethyl (Z)-2-methoxyimino-3-oxobutanoate, **characterized by** without using an acid scavenger and a phase transfer catalyst, and comprising:
mixing ethyl 2-(hydroxyimino)acetoacetate, methanol, a concentrated aqueous sulfuric acid solution, and a boron trifluoride-methanol complex, and subjecting a resulting mixture to esterification-methylation reaction to obtain the ethyl (Z)-2-methoxyimino-3-oxobutanoate.

2. The method as claimed in claim 1, **characterized in that**, the mixing comprises:
subjecting the ethyl 2-(hydroxyimino)acetoacetate and the methanol to primary stirring to obtain a primary mixed solution; and
dropwise adding the concentrated aqueous sulfuric acid solution and the boron trifluoride-methanol complex to the primary mixed solution.

3. The method as claimed in claim 2, **characterized in that**, the concentrated aqueous sulfuric acid solution is added dropwise at a dripping rate of 20-30 g/h, and the boron trifluoride-methanol complex is added dropwise at a dripping rate of 6-10 g/h; and
dropwise adding the concentrated aqueous sulfuric acid solution and the boron trifluoride-methanol complex is performed by starting simultaneously dripping addition of the concentrated aqueous sulfuric acid solution and the boron trifluoride-methanol complex.

4. The method as claimed in any one of claims 1 to 3, **characterized in that**, the concentrated aqueous sulfuric acid solution has a concentration of 95-98 wt%;
a molar ratio of sulfuric acid in the concentrated aqueous sulfuric acid solution to the ethyl 2-(hydroxyimino)acetoacetate ranges from 0.9 : 1 to 2 : 1; and
a molar ratio of the ethyl 2-(hydroxyimino)acetoacetate, the methanol, and the boron trifluoride-methanol complex is in a range of 1 : 10-15 : 0.2-0.5.

5. The method as claimed in claim 1, **characterized in that**, the esterification-methylation reaction is conducted at a temperature of 20-40 °C for 2-4 hours.

6. The method as claimed in claim 1 or 5, **characterized in that**, the esterification-methylation reaction is conducted under stirring, and the stirring is conducted at a stirring speed of 90-150 r/min.

7. The method as claimed in claim 1 or 5, **characterized in that**, the method further comprises: after the esterification-methylation reaction,
mixing a reaction mixture obtained from the esterification-methylation reaction with a saturated sodium carbonate solution, subjecting a resulting liquid mixture to a first separation for removing methanol, collecting a residue obtained from the first separation and subjecting the residue to extraction with dichloromethane, and collecting an organic phase obtained from the extraction and subjecting the organic phase to a second separation for removing dichloromethane to obtain the ethyl (Z)-2-methoxyimino-3-oxobutanoate.

8. The method as claimed in claim 7, **characterized in that**, the first separation is performed by distillation, and the distillation is conducted at a temperature of 40-50 °C; and
the method further comprises: after the distillation, collecting the methanol removed by the distillation, and reusing the methanol in the esterification-methylation reaction.

9. The method as claimed in claim 7, **characterized in that**, the second separation is performed by concentration, the concentration being reduced-pressure concentration; and
the method further comprises: after the concentration, collecting the dichloromethane removed by the concentration, and reusing the dichloromethane in the extraction.

10. An apparatus for preparing ethyl (Z)-2-methoxyimino-3-oxobutanoate, **characterized by** comprising:
a methanol storage tank (4);
a dichloromethane storage tank (5);
an esterification-methylation reactor (1), a first separation device (2), and a second separation device (3), communicated sequentially, wherein
the esterification-methylation reactor (1) is provided with an ethyl 2-(hydroxyimino)acetoacetate feed inlet, a methanol feed inlet, a concentrated aqueous sulfuric acid solution feed inlet, a boron trifluoride-methanol complex feed inlet, and a reaction liquid discharge outlet;
the first separation device (2) is provided with a saturated sodium carbonate solution feed inlet, a dichloromethane feed inlet, a methanol discharge outlet, an organic phase discharge outlet, and a reaction liquid feed inlet that is communicated with the reaction liquid discharge outlet of the esterification-methylation reactor (1) through a pipeline;
the second separation device (3) is provided with a product discharge outlet, a dichloromethane discharge outlet, and an organic phase feed inlet that is communicated with the organic phase discharge outlet of the first separation device (2) through a pipeline;
the methanol storage tank (4) is provided with a feed inlet and a discharge outlet, the feed inlet of the methanol storage tank (4) is communicated with the methanol discharge outlet of the first separation device (2) through a pipeline, and the discharge outlet of the methanol storage tank (4) is communicated with the methanol feed inlet of the esterification-methylation reactor (1) through a pipeline; and
the dichloromethane storage tank (5) is provided with an inlet and an outlet, the inlet of the dichloromethane storage tank (5) is communicated with the dichloromethane discharge outlet of the second separation device (3) through a pipeline, and the outlet of the dichloromethane storage tank (5) is communicated with the dichloromethane feed inlet of the first separation device (2).
